## Europäisches Patentamt

## European Patent Office

## Office européen des brevets

(19)

(11) Veröffentlichungsnummer. **0 035 132**
**B1**

(12)

# EUROPÄISCHE PATENTSCHRIFT

(45) Veröffentlichungstag der Patentschrift:
19.06.85

(51) Int. Cl.⁴: **A 61 K 9/00**, A 61 K 9/08

(21) Anmeldenummer: 81100862.2

(22) Anmeldetag: 06.02.81

(54) Pharmazeutische Zubereitungen mit Glycerin-alkyläthern und Verwendung der letzteren als pharmazeutische Lösungsmittel.

(30) Priorität: 07.02.80 CH 983/80

(43) Veröffentlichungstag der Anmeldung:
09.09.81 Patentblatt 81/36

(45) Bekanntmachung des Hinweises auf die Patenterteilung:
19.06.85 Patentblatt 85/25

(84) Benannte Vertragsstaaten:
BE CH DE FR GB IT LI LU NL SE

(56) Entgegenhaltungen:
DE - A - 2 058 826
DE - A - 2 252 637
FR - A - 2 381 523
US - A - 1 752 305

"I. Pharmacol. Exp. Therap." Bd. 97 (1949) S. 414-419
"Organisch-Chemische Arzneimittel und ihre Synonyma" Nr. 299, 885, 1013, 1380, 1381, 1732, 1785, 2043, 2299, 2338, 2502, 3197, 3223, 4792, 5058, 6443, 6510

Die Akte enthält technische Angaben, die nach dem Eingang der Anmeldung eingereicht wurden und die nicht in dieser Patentschrift enthalten sind.

(73) Patentinhaber: F. HOFFMANN-LA ROCHE & CO. Aktiengesellschaft, CH-4002 Basel (CH)

(72) Erfinder: Boguth, Prof. Dr. Walter, Sonneggstrasse 17, CH-4125 Riehen (CH)
Erfinder: Hirth, Georges, 7, rue de L'ancre, F-68330 Huningue (FR)

(74) Vertreter: Lederer, Franz, Dr. et al, Patentanwälte Dr. Franz Lederer Reiner F. Meyer Lucile-Grahn-Strasse 22, D-8000 München 80 (DE)

## Beschreibung

Die Erfindung betrifft Injektionslösungen, die einen Glycerin-nieder-alkyläther, nämlich Glycerin-1,2-dimethyläther, Glycerin-1,3-dimethyläther, Glycerin-1-methyläther oder Glycerin-1-äthyläther als Lösungsmittel enthalten. Die Erfindung betrifft weiterhin diese Glycerin-nieder-alkyläther zur Verwendung als Lösungsmittel in Injektionslösungen. Die bisher für pharmazeutische Zubereitungen vorgeschlagenen und verwendeten ähnlichen Lösungsmittel sind hinsichtlich verschiedener Eigenschaften, z. B. des Lösungsvermögens, der Mischbarkeit mit anderen Lösungsmitteln und insbesondere der physiologischen Verträglichkeit nicht voll befriedigend. Die Nachteile derartiger handelsüblicher pharmazeutischer Lösungsmittel sind z. B. in der deutschen Auslegeschrift 2 708 419 dargestellt. In der genannten Auslegeschrift werden die vorteilhaften Eigenschaften des 1,2-Butandiol-1-methyläthers als Lösungsmittel für pharmazeutische Zwecke beschrieben. Es hat sich indessen herausgestellt, daß diese Verbindung Veränderungen im Blut hervorruft.

In der US-Patentschrift 1 752 305 werden Glycerinäther, insbesondere Glycerin-diäthyläther, als pharmazeutische Lösungsmittel vorgeschlagen. In der Offenlegungsschrift 2 252 637 werden 1,3-Dialkylglycerinäther als choleretisch wirksame Arzneimittel beschrieben. In M. Negwer: Organisch-chemische Arzneimittel und ihre Synonyme, Akademieverlag (1978) sind zahlreiche pharmakologisch wirksame Glycerinäther, z. B. das 1,3-Dimethoxy-2-propanolnitrat und der Glycerin-1-äthyl-3-isopropyl-diäther erwähnt. In J. Pharmacol Exp. Therap., Bd. 97 (1949), S. 414—419, wird über geringe Muskel-paralysierende Wirkungen u. a. bei Glycerin-1,3-dimethyläther berichtet.

Es wurde nun gefunden, daß die erwähnten Glycerin-nieder-alkyläther besonders geeignete Lösungsmittel für Injektionslösungen darstellen, da sie ein sehr gutes Lösungsvermögen aufweisen, mit andern pharmazeutischen Lösungsmitteln mischbar sind und physiologisch besser verträglich sind als der 1,2-Butandiol-1-methyläther.

Bevorzugt sind der Glycerin-1-methyläther, der Glycerin-1,3-dimethyläther und insbesondere der Glycerin-1,2-dimethyläther.

Die erfindungsgemäßen Injektionslösungen können Wirkstoffe wie Sulfonamide, z. B. Sulfamethoxazol, Sulfadoxin, Sulfamoxol, Sulfathiazin; zweckmäßig in Form verträglicher Salze; oder Sulfonamidpotentiatoren, z. B. Trimethoprim oder Diaveridin; oder Benzodiazepine, z. B. Diazepam, Oxazepam oder Chlordiazepoxid; oder Vitamine, z. B. Vitamin A-Acetat, Vitamin $K_1$ oder Vitamin E enthalten. Von besonderem Interesse sind pharmazeutische Zubereitungen, die Sulfamethoxazol, Trimethoprim und Glycerin-1-methyläther oder Diazepam und Glycerin-1,2-dimethyläther enthalten.

Die erfindungsgemäßen Injektionslösungen können weitere physiologisch unbedenkliche Lösungsmittel, z. B. Wasser oder physiologische Kochsalzlösung, enthalten. Von besonderem Interesse sind pharmazeutische Zubereitungen, die ein Gemisch aus 50—80% Glycerin-nieder-alkyläther und 50—20% Wasser enthalten. Die erfindungsgemäßen pharmazeutischen Zubereitungen enthalten im allgemeinen 10—99, vorzugsweise 40—99 Gew.-% eines Glycerinalkyläthers.

Die Lösungseigenschaften der erfindungsgemäßen Injektionslösungen können gegebenenfalls durch einen Zusatz von Äthanol, z. B. 5—10% Äthanol, verbessert werden. Weiterhin wurde gefunden, daß bei Zubereitungen mit höherem Wassergehalt, die einen in Wasser schwer löslichen Wirkstoff enthalten, die Stabilität bei der Lagerung verbessert werden kann, wenn die Zubereitungen kurzzeitig, z. B. 10 Minuten auf 120°, erhitzt werden.

Die erfindungsgemäßen Injektionslösungen können dadurch erhalten werden, daß man einen pharmazeutischen Wirkstoff in einem der erwähnten Glycerin-nieder-alkyläther, gegebenenfalls unter Zusatz von Wasser, oder Wasser und Äthanol löst.

Die erfindungsgemäßen Injektionslösungen können für intravenöse oder intramuskuläre Verabreichung von Wirkstoffen dienen.

Die folgenden Beispiele sollen die Erfindung weiter erläutern.

### Beispiel 1

1 g Sulfamethoxazol wird unter schwachem Erwärmen in 1 ml 4N Natronlauge gelöst. Zu dieser Lösung werden unter Rühren 0,2 g Trimethoprim in 3,5 ml Glycerin-1-methyläther gegeben, wobei eine klare Lösung resultiert. Die Lösung wird gegebenenfalls steril filtriert und in Ampullen gefüllt.

### Beispiel 2

10 mg Diazepam werden in 1,2 ml Glycerin-1,2-dimethyläther gelöst. Die Lösung wird mit 0,8 ml Wasser versetzt, gegebenenfalls steril filtriert und in Ampullen gefüllt.

### Beispiel 3

10 mg Diazepam werden in 1,5 ml Glycerin-1-methyläther gelöst. Die Lösung wird mit 0,5 ml Wasser vermischt, gegebenenfalls steril filtriert und in Ampullen gefüllt.

### Beispiel 4

10 mg Diazepam werden in 1,2 ml Glycerin-1-äthyläther gelöst. Die Lösung wird mit 0,8 ml Wasser vermischt, gegebenenfalls steril filtriert und in Ampullen gefüllt.

### Beispiel 5

15 mg Vitamin A-Acetat werden in 1 ml Glycerin-1-methyläther gelöst. Die Lösung wird gegebenenfalls steril filtriert und in Ampullen gefüllt.

### Beispiel 6

200 mg Vitamin $K_1$ werden in 1 ml Glycerin-1,2-dimethyläther gelöst. Die Lösung wird gegebenenfalls steril filtriert und in Ampullen gefüllt.

### Beispiel 7

200 mg Vitamin E werden in 1 ml Glycerin-1,2-dimethyläther gelöst. Die Lösung wird gegebenenfalls steril filtriert und in Ampullen gefüllt.

### Beispiel 8

1 g Sulfamethoxazol wird unter schwachem Erwärmen in 1 ml 4N Natronlauge gelöst. Zu dieser Lösung werden unter gutem Durchmischen 0,2 g Trimethoprim in 3,05 ml Glycerin-1-methyläther und 0,45 ml Feinsprit gegeben, wobei eine klare Lösung resultiert.

### Beispiel 9

1 g Sulfadoxin wird unter schwachem Erwärmen in 1 ml 3,3N Natronlauge gelöst. Zu dieser Lösung werden unter gutem Durchmischen 0,2 g Trimethoprim in 3,05 ml Glycerin-1-methyläther und 0,45 ml Feinsprit gegeben, wobei eine klare Lösung resultiert.

### Beispiel 10

10 mg Diazepam werden in 0,9 ml Glycerin-1,2-dimethyläther und 0,1 ml Feinsprit gelöst und unter gutem Durchmischen mit 1 ml physiologischer Kochsalzlösung versetzt. Man erhält eine klare Lösung.

### Beispiel 11

10 mg Diazepam werden in 0,8 ml Glycerin-1,3 dimethyläther und 0,1 ml Feinsprit gelöst und unter gutem Durchmischen mit 1,1 ml physiologischer Kochsalzlösung versetzt. Man erhält eine klare Lösung.

### Beispiel 12

10 mg Diazepam werden in 0,9 ml Glycerin-1-äthyläther und 0,1 ml Feinsprit gelöst und mit 1,0 ml physiologischer Kochsalzlösung zu einer klaren Lösung vereint.

### Beispiel 13

10 mg Diazepam werden in 1,0 ml Glycerin-1-methyläther und 0,1 ml Feinsprit gelöst und mit 0,9 ml physiologischer Kochsalzlösung zu einer klaren Lösung vermischt.

### Beispiel 14

15 mg Vitamin A-acetat werden in 1 ml Glycerin-1-methyläther bei Raumtemperatur klar gelöst.

### Beispiel 15

200 mg Vitamin $K_1$ werden in 1 ml Glycerin-1,2-dimethyläther gelöst, wobei eine klare Lösung resultiert.

### Beispiel 16

200 mg Tocopherol werden in 1 ml Glycerin-1,2-dimethyläther gelöst, wobei eine klare Lösung resultiert.

### Patentansprüche

1. Injektionslösungen, die als Lösungsmittel Glycerin-1,2-dimethyläther, Glycerin-1,3-dimethyläther, Glycerin-1-methyläther oder Glycerin-1-äthyläther enthalten.

2. Injektionslösungen gemäß Anspruch 1, die als Lösungsmittel ein Gemisch von 50—80% Glycerinäther und 50—20% Wasser enthalten.

3. Injektionslösungen gemäß den Ansprüchen 1 oder 2, die als weiterer Bestandteil Äthanol enthalten.

4. Injektionslösungen gemäß den Ansprüchen 1—3, die als Wirkstoff ein Sulfonamid, ein Benzodiazepin oder ein Vitamin enthalten.

5. Injektionslösungen gemäß den Ansprüchen 1—4, enthaltend Sulfamethoxazol, Trimethoprim und Glycerin-1-methyläther.

6. Injektionslösungen gemäß den Ansprüchen 1—4, enthaltend Diazepam und Glycerin-1,2-dimethyläther.

7. Glycerin-1,2-dimethyläther, Glycerin-1,3-dimethyläther, Glycerin-1-methyläther oder Glycerin-1-äthyläther zur Verwendung als Lösungsmittel in Injektionslösungen.

### Claims

1. Injection solutions which contain glycerol 1,2-dimethyl ether, glycerol 1,3-dimethyl ether, glycerol 1-methyl ether or glycerol 1-ethyl ether as the solvent.

2. Injection solutions in accordance with claim 1, which contain a mixture of 50—80% glycerol ether and 50—20% water as the solvent.

3. Injection solutions in accordance with claims 1 or 2, which contain ethanol as a further ingredient.

4. Injection solutions in accordance with claims 1—3, which contain a sulphonamide, a benzodiazepine or a vitamin as the active substance.

5. Injection solutions in accordance with claims 1—4, containing sulphamethoxazole, trimethoprim and glycerol 1-methyl ether.

6. Injection solutions in accordance with claims 1—4, containing diazepam and glycerol 1,2-dimethyl ether.

7. Glycerol 1,2-dimethyl ether, glycerol 1,3-dimethyl ether, glycerol 1-methyl ether or glycerol 1-ethyl ether for use as solvents in injection solutions.

**Revendications**

1. Solutions pour injections contenant en tant que solvant l'éther 1,2-diméthylique du glycérol, l'éther 1,3-diméthylique du glycérol, l'éther 1-méthylique du glycérol ou l'éther 1-éthylique du glycérol.

2. Solutions pour injections selon la revendication 1, contenant en tant que solvant un mélange de 50 à 80% d'éther du glycérol et de 50 à 20% d'eau.

3. Solutions pour injections selon les revendications 1 ou 2, contenant de l'éthanol en tant qu'autre constituant.

4. Solutions pour injections selon les revendications 1 à 3, contenant en tant que substance active un sulfonamide, une benzodiazépine ou une vitamine.

5. Solutions pour injections selon les revendications 1 à 4, contenant du Sulfamethoxazol, du Trimethoprim et de l'éther 1-méthylique du glycérol.

6. Solutions pour injections selon les revendications 1 à 4, contenant du Diazepam et de l'éther 1,2-diméthylique du glycérol.

7. L'éther 1,2-diméthylique du glycérol, l'éther 1,3-diméthylique du glycérol, l'éther 1-méthylique du glycérol, ou l'éther 1-éthylique du glycérol pour l'utilisation en tant que solvant dans des solutions pour injections.